# EUROPEAN PATENT APPLICATION

(11) **EP 4 737 564 A1**
(43) Date of publication of application: **06.05.2026**
(21) Application number: 24830120.2
(22) Date of filing: 26.04.2024
(51) Int. Cl.: C12N 9/10, C12N 15/54, C12N 15/70, C12N 1/21, C12P 19/56, C12P 19/18

(54) **GLYCOSYLTRANSFERASE UGTSL2 MUTANT, GLYCOSYLTRANSFERASE MUTANT, AND METHOD FOR SYNTHESIZING REBAUDIOSIDE M2 THEREWITH**

(30) Priority: 30.06.2023 CN 202310795002; 30.06.2023 CN 202310813254
(71) Applicant: Dongtai Haorui Biotechnology Co., Ltd, Yancheng, Jiangsu 224237 (CN)
(72) Inventor: ZHU, Liping, Yancheng, Jiangsu 224237 (CN); JIA, Honghua, Yancheng, Jiangsu 224237 (CN); LI, Yan, Yancheng, Jiangsu 224237 (CN); CHEN, Kecai, Yancheng, Jiangsu 224237 (CN); PAN, Huayi, Yancheng, Jiangsu 224237 (CN); SONG, Weicai, Yancheng, Jiangsu 224237 (CN); CHEN, Kai, Yancheng, Jiangsu 224237 (CN); XU, Liangping, Yancheng, Jiangsu 224237 (CN); SHEN, Yingying, Yancheng, Jiangsu 224237 (CN)
(74) Representative: Metida
(86) International application number: PCT/CN2024/090169
(87) International publication number: WO 2025/001471

(57) **Abstract**

Provided is a glycosyltransferase UGTSL2 mutant . Said UGTSL2 mutant is obtained by means of any one or more of the following mutations on an amino acid sequence shown as SEQ ID NO .1:the 23rd amino acid is mutated from N to E ; the 41st amino acid is mutated from R to P ; the 91st amino acid is mutated from H to K : the 95th amino acid is mutated from K to D ; the 123rd amino acid is mutated from E to P ; the 136th amino acid is mutated from L to F : the 151st amino acid is mutated from R to F ; the 124th amino acid is mutated from H to E ; the 168th amino acid is mutated from V to Y ;the 198th amino acid is mutated from C to K ; the 202nd amino acid is mutated from T to E ; the 217th amino acid is mutated from W to K : the 225th amino acid is mutated from P to L ; the 226th amino acid is mutated from F to V ; the 285th amino acid is mutated from A to V ; the 333rd amino acid is mutated from I to V , etc . Also provided is a glycosyltransferase mutant , the glycosyltransferase mutant being following A1) or A2):A1) being a protein which is obtained by means of substitution of an amino acid residue on an amino acid sequence shown as SEQ ID NO .66, and which has 90% or higher identity with and same functions as the original protein , and A2) being a fusion protein obtained by linking a tag to the Nterminal and / or the Cterminal of A1.

## Description

### CROSS REFERENCE TO RELATED APPLICATIONS

The present application claims priority to the Chinese Patent 202310795002.3 filed to the China National Intellectual Property Administration (CNIPA) on June 30, 2023 and entitled "GLYCOSYLTRANSFERASE MUTANT AND METHOD FOR SYNTHESIZING REBAUDIOSIDE M2 UNDER CATALYSIS OF MUTANT" and the Chinese Patent 202310813254.4 filed to the China National Intellectual Property Administration (CNIPA) on June 30, 2023 and entitled "GLYCOSYLTRANSFERASE UGTSL2 MUTANT AND METHOD FOR SYNTHESIZING REBAUDIOSIDE M2", which are incorporated herein by reference in their entireties.

### TECHNICAL FIELD

The present disclosure relates to the technical field of biocatalytic synthesis, and specifically to a glycosyltransferase UGTSL2 mutant, a glycosyltransferase mutant, and a method for synthesizing rebaudioside M2 under catalysis of the mutants.

### BACKGROUND

There is a growing demand for zero-calorie natural sugar substitutes, especially by diabetics. Steviol glycosides (SGs) are a group of natural calorie-free ingredients extracted from the leaves of the stevia plant. SGs have been approved as safe food additives in countries such as the United States, European countries, and Asian countries. In 2014, the use of SGs as food additives in products such as food, beverages, and nutritional supplements was approved in China. Therefore, SGs have received extensive attention.

Rebaudioside D is a very promising SG with sweetness 200 times to 300 times higher than sucrose. However, the short-term lingering bitterness carried by rebaudioside D affects the application and promotion of rebaudioside D on the market. Rebaudioside M2 is produced by adding a glucosyl group at C-6' of the first glucosyl group linked to C19 of rebaudioside D. Rebaudioside M2 exhibits increased sweetness and no bitterness compared with rebaudioside D. Thus, rebaudioside M2 has better sugar properties and a more desirable taste than rebaudioside D.

Currently, rebaudioside M2 is a monoglycosylated derivative of rebaudioside D, and is prepared through biotransformation. NtUGT derived from *Nicotiana tomentosiformis* can catalyze the C-6' glycosylation of the first glucosyl group linked to C19 of an SG compound, such that the high-value sweetener rebaudioside M2 can be produced by catalyzing rebaudioside D with *Nt*UGT.

*Nt*UGT is a Leloir glycosyltransferase belonging to the GT1 family. Glycosides are generally synthesized with uridine diphosphate glucose (UDPG) as a glycosyl donor. *Nt*UGT can be produced in large quantities through the heterologous expression in *Escherichia coli* or *Saccharomyces cerevisiae.* As a result, *Nt*UGT can be used to catalyze the synthesis of rebaudioside M2 from rebaudioside D. With the continuous improvement of UDPG production methods, UDPG can be synthesized in large quantities with enzymes such as sucrose synthase (SuSy). On this basis, the SuSy-UGT cascade reaction system can be adopted. In the SuSy-UGT cascade reaction system, SuSy provides as a glycosyl donor for NtUGT to achieve the recycling of UDPG, which greatly reduces the application cost. In the NtUGT-SuSy cascade reaction system, SuSy provides a glycosyl donor for the smooth glycosylation. When a regeneration rate of UDPG is not limited (for example, the regeneration rate of UDPG can be increased by increasing a sucrose concentration), *Nt*UGT is the key to the synthesis efficiency of rebaudioside M2. The tobacco-derived *Nt*UGT exhibits poor thermal stability and is basically inactivated after 10 h of catalysis, which limits the efficient application of the glycosyltransferase.

### SUMMARY

Therefore, a technical problem to be solved by the present disclosure is to overcome the defects of a low enzyme activity and a short half-life period of the glycosyltransferase UGTSL2 in the prior art and the defect of poor thermal stability of NtUGT and thus provide a glycosyltransferase UGTSL2 mutant with high enzyme activity and long half-life and a glycosyltransferase mutant with high M2 yield and strong thermal stability.

In a first aspect, the present disclosure provides a glycosyltransferase UGTSL2 mutant, where the glycosyltransferase UGTSL2 mutant is any one selected from the group consisting of the following (A) to (C):
(A) a protein produced through any one or more selected from the group consisting of the following mutations based on an amino acid sequence shown in SEQ ID NO: 1:
   a mutation of a 23rd amino acid from N to E;
   a mutation of a 41st amino acid from R to P;
   a mutation of a 91st amino acid from H to K;
   a mutation of a 95th amino acid from K to D;
   a mutation of a 123rd amino acid from E to P;
   a mutation of a 136th amino acid from L to F;
   a mutation of a 151st amino acid from R to F;
   a mutation of a 124th amino acid from H to E;
   a mutation of a 168th amino acid from V to Y;
   a mutation of a 198th amino acid from C to K;
   a mutation of a 202nd amino acid from T to E;
   a mutation of a 217th amino acid from W to K;
   a mutation of a 225th amino acid from P to L;
   a mutation of a 226th amino acid from F to V;
   a mutation of a 285th amino acid from A to V;
   a mutation of a 333rd amino acid from I to V;
   a mutation of a 358th amino acid from N to F;
   a mutation of a 392nd amino acid from T to V; and
   a mutation of a 419th amino acid from I to K;
(B) a protein that has an identity of 95% or more with and the same function as an amino acid sequence defined in the (A); and
(C) a fusion protein produced by linking a tag to a terminus of the protein defined in the (A) or the (B).

Preferably, an amino acid sequence of the glycosyltransferase UGTSL2 mutant is shown in SEQ ID NO: 3.

The present disclosure provides a biological material selected from the group consisting of the following:
(A) an expression gene encoding the glycosyltransferase UGTSL2 mutant according to claim 1 or 2;
(B) a recombinant plasmid carrying the expression gene in the (A); and
(C) a recombinant cell carrying the recombinant plasmid or the expression gene encoding the glycosyltransferase UGTSL2 mutant.

The expression gene is produced through one or more selected from the group consisting of the following mutations based on SEQ ID NO: 2: a substitution of AAC at positions 69 to 71 in SEQ ID NO: 2 with GAA;
a substitution of CGT at positions 121 to 123 in SEQ ID NO: 2 with CCG;
a substitution of CAC at positions 271 to 273 in SEQ ID NO: 2 with AAA;
a substitution of AAA at positions 283 to 285 in SEQ ID NO: 2 with GAT;
a substitution of GAG at positions 367 to 369 in SEQ ID NO: 2 with CCG;
a substitution of CTG at positions 406 to 408 in SEQ ID NO: 2 with TTT;
a substitution of CGT at positions 451 to 453 in SEQ ID NO: 2 with TTT;
a substitution of CAC at positions 370 to 372 in SEQ ID NO: 2 with GAA;
a substitution of GTG at positions 502 to 504 in SEQ ID NO: 2 with TAT;
a substitution of TGC at positions 592 to 594 in SEQ ID NO: 2 with AAA;
a substitution of ACC at positions 604 to 606 in SEQ ID NO: 2 with GAA;
a substitution of TGG at positions 649 to 651 in SEQ ID NO: 2 with AAA;
a substitution of CCG at positions 673 to 675 in SEQ ID NO: 2 with CTG;
a substitution of TTC at positions 676 to 678 in SEQ ID NO: 2 with GTT;
a substitution of GCG at positions 853 to 855 in SEQ ID NO: 2 with GTT;
a substitution of ATT at positions 997 to 999 in SEQ ID NO: 2 with GTT;
a substitution of AAC at positions 1,072 to 1,074 in SEQ ID NO: 2 with TTC;
a substitution of ACC at positions 1,174 to 1,176 in SEQ ID NO: 2 with GTT; and
a substitution of ATT at positions 1,255 to 1,257 in SEQ ID NO: 2 with AAA.

Preferably, a nucleotide sequence of the expression gene is shown in SEQ ID NO: 4.

The present disclosure provides an enzyme composition, including: a glycosyltransferase UGTSL2 mutant, a glycosyltransferase *Nt*UGT_M, and SuSy AtSuSy, where the glycosyltransferase UGTSL2 mutant is the glycosyltransferase UGTSL2 mutant according to claim 1 or 2;
the glycosyltransferase *Nt*UGT_M is the following (A1) or (A2) :(A1) an amino acid sequence shown in SEQ ID NO: 5; and
(A2) a protein that has an identity of 95% or more with and the same function as the amino acid sequence defined in the (A1); and
the SuSy AtSuSy is the following (B1) or (B2):(B1) an amino acid sequence shown in SEQ ID NO: 7; and
(B2) a protein that has an identity of 95% or more with and the same function as the amino acid sequence defined in the (B1).

Preferably, an enzyme activity ratio of the glycosyltransferase UGTSL2 mutant to the glycosyltransferase *Nt*UGT_M is (3-7):(7-3).

The present disclosure provides a complete set of recombinant strains expressing the enzyme composition described above, including a recombinant strain A and a recombinant strain B, where the recombinant strain A includes a recombinant plasmid A, and the recombinant plasmid A is produced by co-constructing a nucleic acid encoding the glycosyltransferase UGTSL2 mutant according to claim 1 or 2 and a nucleic acid encoding the SuSy *At*SuSy into an expression vector; the recombinant strain B includes a recombinant plasmid B, and the recombinant plasmid B is produced by co-constructing a nucleic acid encoding the glycosyltransferase *Nt*UGT_M and a nucleic acid encoding the SuSy *At*SuSy into a plasmid; and an amino acid sequence of the glycosyltransferase *Nt*UGT_M is shown in SEQ ID NO: 5.

Host strains include, but are not limited to, *Escherichia coli, Saccharomyces cerevisiae, Pichia pastoris,* or *Corynebacterium glutamicum.*

The present disclosure provides a method for one-pot synthesis of rebaudioside M2 from rebaudioside A under biocatalysis, including the following steps:
adding the rebaudioside A, sucrose, and the enzyme composition described above or an induced expressed enzyme product of the complete set of recombinant strains according to claim 5 to a catalytic reaction system, allowing a reaction, and conducting enzyme inactivation and centrifugation to produce a supernatant, which is the rebaudioside M2, where the rebaudioside M2 is shown in the following formula (I):

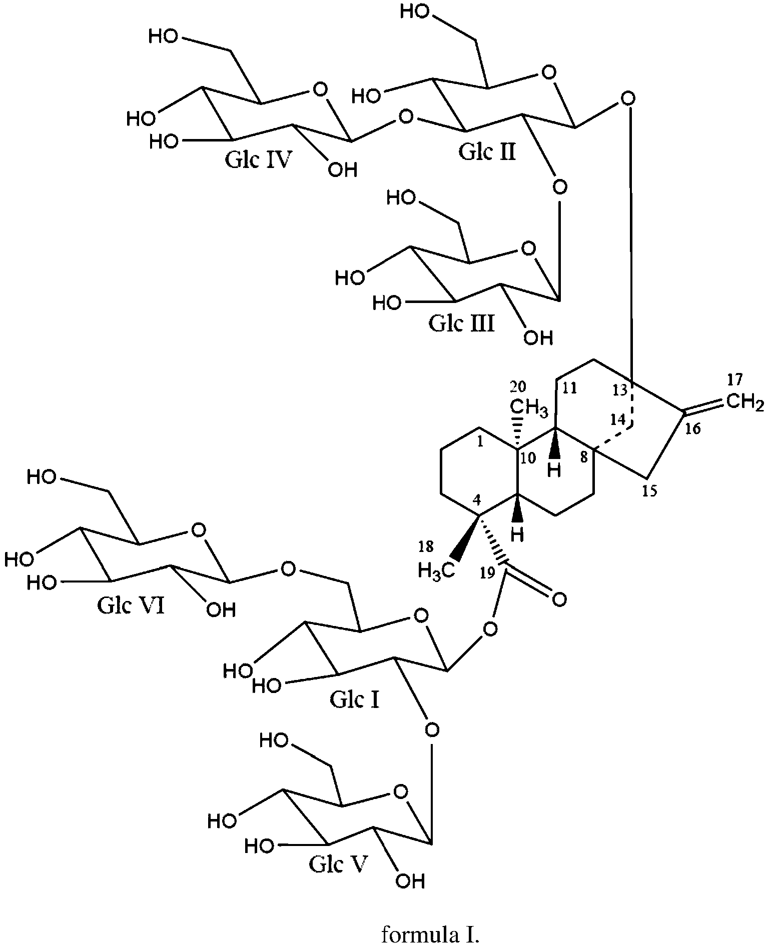

Preferably, a process for acquiring the induced expressed enzyme product of the complete set of recombinant strains described above includes the following step: 1) activating the complete set of recombinant strains described above to produce activated recombinant strains, transferring the activated recombinant strains to an induction medium, adding an inducer, and conducting an induction culture; conducting centrifugation to collect strain cells, resuspending the strain cells in an appropriate amount of a buffer, and conducting disruption; and conducting centrifugation to produce a supernatant, which is the induced expressed enzyme product; and the reaction is conducted for 5 h to 100 h.

Preferably, a final concentration of the inducer is 0.02 g/L to 1 g/L, and the induction culture is conducted for 4 h to 50 h.

Preferably, in the catalytic reaction system, a concentration of the rebaudioside A is 1 g/L to 1,000 g/L, a concentration of the sucrose is 1 g/L to 9,000 g/L, and an amount of the induced expressed enzyme product added is 1 MU/L to 100 MU/L; a pH of the catalytic reaction system is 7.0 to 8.5 and preferably 7.0; the reaction is conducted at 37°C to 40°C; and an enzyme activity ratio of the glycosyltransferase UGTSL2 mutant to the glycosyltransferase *Nt*UGT_M is (3-7):(7-3).

The catalytic reaction system also includes uridine diphosphate (UDP) of 1 mM to 4 mM and preferably 3 mM.

The present disclosure has the following advantages:
(1) The mutant has a high enzyme activity and a long half-life period.
(2) The method for one-pot synthesis of rebaudioside M2 from rebaudioside A under biocatalysis in the present application achieves the short-time efficient catalysis of synthesis of rebaudioside M2 from rebaudioside A. Moreover, the biological enzyme catalysis method is eco-friendly and pollution-free, and is suitable for the green industrial processing and production.
(3) The method for one-pot synthesis of rebaudioside M2 from rebaudioside A under biocatalysis can achieve the regeneration of UDPG *in vivo* by constructing a double-enzyme system, which effectively provides a source for expensive glycosyl donors, reduces the cost, and promotes the application of biotechnology industry.

In conclusion, in the present application, through the consensus strategy, the protein molecular dynamics simulation, and the computer-simulated screening technology, a site-directed mutation library for the glycosyltransferase UGTSL2 is established based on a structure, and the glycosyltransferase UGTSL2 is modified. The combination of a glycosyltransferase and SuSy achieves the recycling of the substrate UDPG as a glycosyl donor with cheap sucrose. In addition, a cascade reaction system of "one pot + three enzymes" is established to synthesize rebaudioside M2 from rebaudioside A, a cheap raw material.

In a second aspect, the present application provides a glycosyltransferase mutant, which is the following A1) or A2):
A1) a protein produced through an amino acid residue substitution in an amino acid sequence shown in SEQ ID NO: 66, where the protein has an identity of 90% or more with and the same function as the amino acid sequence shown in SEQ ID NO: 66; and
A2) a fusion protein produced by linking a tag to an N-terminus and/or a C-terminus of the protein in the A1).

Preferably, the glycosyltransferase mutant is a protein produced through the following one or more amino acid residue substitutions in the amino acid sequence shown in SEQ ID NO: 66:
B1) a mutation of a 10th amino acid from alanine (A) to valine (V);
B2) a mutation of a 51st amino acid from glutamine (Q) to lysine (K);
B3) a mutation of a 72nd amino acid from phenylalanine (F) to leucine (L);
B4) a mutation of a 87th amino acid from leucine (L) to proline (P);
B5) a mutation of a 123rd amino acid from leucine (L) to proline (P);
B6) a mutation of a 157th amino acid from leucine (L) to proline (P);
B7) a mutation of a 219th amino acid from asparagine (N) to aspartic acid (D);
B8) a mutation of a 380th amino acid from glycine (G) to leucine (L); and
B9) a mutation of a 400th amino acid from serine (S) to arginine (R).

Preferably, the glycosyltransferase mutant has an amino acid sequence produced through the above mutations other than the mutation in the B9) based on SEQ ID NO: 66.

The present disclosure provides a biological material, which is any one selected from the group consisting of the following C1) to C5):
C1) a nucleic acid encoding the protein;
C2) an expression cassette carrying the nucleic acid in the C1);
C3) a recombinant vector carrying the nucleic acid in the C1) or a recombinant vector carrying the expression cassette in the C2);
C4) a recombinant microorganism carrying the nucleic acid in the C1), or a recombinant microorganism carrying the expression cassette in the C2), or a recombinant microorganism carrying the recombinant vector in the C3); and
C5) a transgenic plant cell line carrying the nucleic acid in the C1) or a transgenic plant cell line carrying the expression cassette in the C2).

The nucleic acid encoding the protein is produced through any one or more selected from the group consisting of the following mutations based on SEQ ID NO: 65:
a substitution of CAG at positions 150 to 152 in SEQ ID NO: 65 with AAG;
a substitution of TTC at positions 216 to 218 in SEQ ID NO: 65 with CTT;
a substitution of CTG at positions 391 to 393 in SEQ ID NO: 65 with CCT;
a substitution of TTG at positions 471 to 473 in SEQ ID NO: 65 with CCT;
a substitution of AAC at positions 657 to 659 in SEQ ID NO: 65 with GAC;
a substitution of GCT at positions 28 to 30 in SEQ ID NO: 65 with GTT;
a substitution of CTG at positions 259 to 261 in SEQ ID NO: 65 with CCT; and
a substitution of GGT at positions 1,138 to 1,140 in SEQ ID NO: 65 with TTA.

Preferably, a nucleotide sequence of the nucleic acid encoding the protein is shown in SEQ ID NO: 69.

The present disclosure also provides a method for synthesizing rebaudioside M2 under catalysis of a glycosyltransferase mutant, including the following steps:
1) construction of a recombinant strain carrying a double-enzyme co-expression system: co-constructing a coding gene for the glycosyltransferase mutant described above and a SuSy gene into an expression vector to produce a recombinant plasmid, and transforming the recombinant plasmid into a host strain to produce the recombinant strain carrying the double-enzyme co-expression system;
2) inducing the recombinant strain to express the glycosyltransferase mutant and SuSy; and
3) using the two enzymes obtained in the step 2) to prepare rebaudioside M2 with rebaudioside D and sucrose as raw materials:

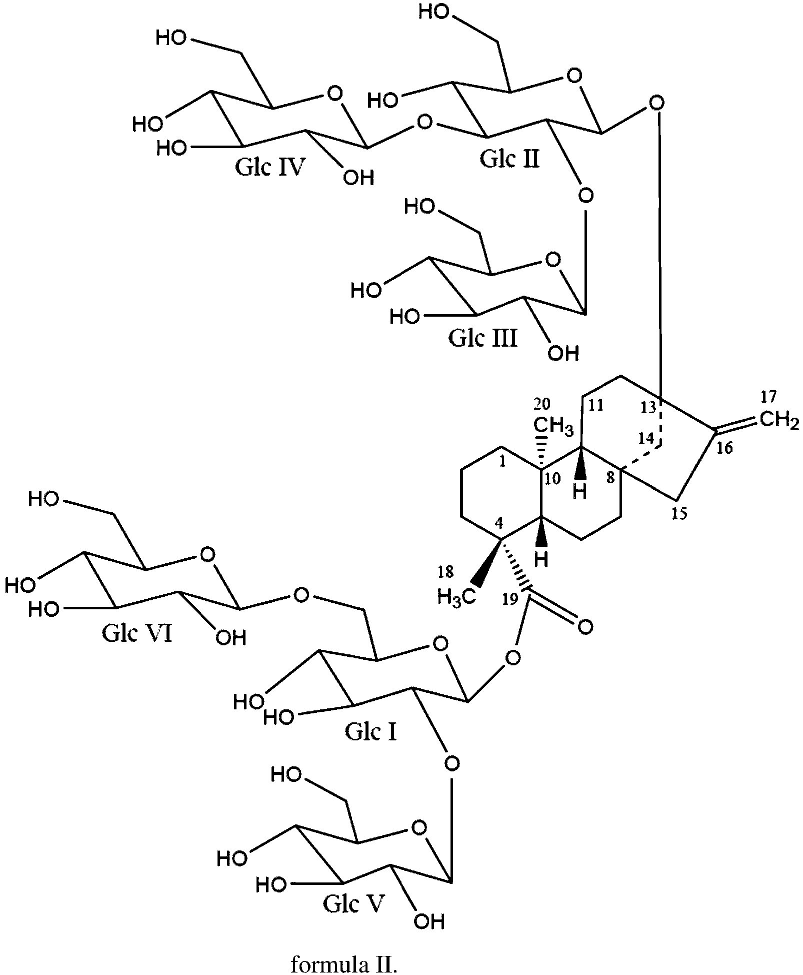

Preferably, the expression vector is pRSFDuet-1.

Preferably, for the induced expression in the step 2), an inducer is added at a final concentration of 0.02 g/L to 1 g/L, and induction is conducted for 4 h to 50 h.

Preferably, the step 2) includes: strain collection, strain disruption, and centrifugation to collect a supernatant, which is a crude enzyme.

Preferably, in the step 3), a concentration of the rebaudioside D is 1 g/L to 500 g/L, a concentration of the sucrose is 1 g/L to 1,500 g/L, and an amount of the crude enzyme added is 1 g/L to 100 g/L.

Preferably, the host strain includes, but is not limited to, *Escherichia coli, Saccharomyces cerevisiae, Pichia pastoris,* or *Corynebacterium glutamicum.*

Preferably, the rebaudioside D is synthesized with rebaudioside A as a substrate under catalysis of a glycosyltransferase.

In the present disclosure, active central residues of the glycosyltransferase NtUGT are subjected to site-directed mutagenesis, and the relative activities and half-life periods of the resulting mutants are compared to obtain the optimal glycosyltransferase mutant NtUGT_A10V/Q51K/F72L/L87P/L123P/L157P/N219D/G380L. The cascade of the glycosyltransferase mutant with SuSy can catalyze the synthesis of rebaudioside M2 with SG as a substrate in combination with an appropriate amount of sucrose. The mutant can be simply prepared, and can achieve the efficient catalytic synthesis of rebaudioside M2. Under the same conditions, the mutant has an activity 5.7 times higher than the original enzyme and a half-life period about 2.9 times longer than the original enzyme, and leads to a significantly higher yield of rebaudioside M2 than the original enzyme. A reaction system based on the mutant NtUGT_A10V/Q51K/F72L/L87P/L123P/L157P/N219D/G380L exhibits a better catalytic effect than a non-mutant system, and can lead to a rebaudioside M2 concentration of 36.7 g/L at 36 h of a reaction and a yield of 95% or more.

Through mutagenesis, the present disclosure improves the thermal stability of the glycosyltransferase NtUGT, prolongs T_{35°C} by about three times, and increases a catalytic activity by about 10 times, thereby improving the efficiency of catalytic production of rebaudioside M2.

The present disclosure improves an enzyme activity of the glycosyltransferase NtUGT through site-directed mutagenesis. The mutant can achieve the efficient catalytic synthesis of rebaudioside M2. The corresponding synthesis method involves mild conditions, simple operations, short time, high catalytic efficiency, and high yield, and has a promising application prospect.

### BRIEF DESCRIPTION OF THE DRAWINGS

To describe the technical solutions in the specific implementations of the present disclosure or the prior art more clearly, the accompanying drawings required for describing the specific implementations or the prior art are briefly described below. Apparently, the accompanying drawings in the following description show merely some implementations of the present disclosure, and a person of ordinary skill in the art may still derive other accompanying drawings from these accompanying drawings without creative efforts.
FIG. 1 shows product yields of a one-pot reaction system during temperature optimization in Example 4 of the present disclosure, where RM21G is a self-named by-product;
FIG. 2 shows product yields of a one-pot reaction system during pH optimization in Example 5 of the present disclosure, where RM21G is a self-named by-product;
FIG. 3 shows product yields of a one-pot reaction system during optimization of an enzyme activity ratio of UGTSL2_M to NtUGT in Example 6 of the present disclosure, where RM21G is a self-named by-product;
FIG. 4 shows product yields of a one-pot reaction system during optimization of an addition proportion of a substrate sucrose in Example 7 of the present disclosure, where RM21G is a self-named by-product;
FIG. 5 shows product yields of a one-pot reaction system during optimization of an addition amount of UDP in Example 8 of the present disclosure, where RM21G is a self-named by-product; and
FIG. 6 shows a structural simulation of an NtUGT protein and a spatial location of a mutant amino acid.

### DETAILED DESCRIPTION OF THE EMBODIMENTS

LB medium: NaCl: 10 g/L, yeast powder: 5 g/L, peptone: 10 g/L, and agar: 20 g/L.

### Example 1 Preparation of a recombinant strain expressing a glycosyltransferase UGTSL mutant and a SuSy AtSuSy

A double-gene expression vector pRSFDuet-1 (this vector was published in Han Guangwei. Research on Co-expression and Immunogenicity of α, β_1, β_2, and ε toxin proteins of *Clostridium perfringens*[*D*]. Chinese Academy of Agricultural Sciences, 2014.) was selected. A nucleotide sequence of a coding gene for a stevia-derived glycosyltransferase UGTSL or a mutant thereof was inserted to an *Nde*I/*Xho*I site of the above vector, and a nucleotide sequence for an *Arabidopsis thaliana-derived* SuSy *At*SuSy was inserted to a *Nco*I/*Eco*RI site of the above vector, so as to produce a recombinant expression plasmid pRSFDuet-1-*At*SuSy-UGTSL. The recombinant plasmid was transformed into *Escherichia coli* BL21 (DE3), and the induced expression was conducted to produce BL21-pRSFDuet-1-AtSuSy-UGTSL. An amino acid sequence of the glycosyltransferase UGTSL was shown in SEQ ID NO: 1. A nucleotide sequence of a coding gene for the glycosyltransferase UGTSL was shown in SEQ ID NO: 2.

The above mutant included the following single-point mutations: UGTSL2_N23E, UGTSL2_R41P, UGTSL2_H91K, UGTSL2_K95D, UGTSL2_E123P, UGTSL2_L136F, UGTSL2_R151F, UGTSL2_H124E, UGTSL2_V168Y, UGTSL2_C198K, UGTSL2_T202E, UGTSL2_W217K, UGTSL2_P225L, UGTSL2_F226V, UGTSL2_A285V, UGTSL2_I333V, UGTSL2_N358F, UGTSL2_T392V, and UGTSL2_I419K.

N23E was produced through a mutation of a 23rd amino acid in SEQ ID NO: 1 from N to E, and a nucleic acid encoding the protein mutant was produced through a substitution of AAC at positions 69 to 71 in SEQ ID NO: 2 with GAA.

R41P was produced through a mutation of a 41st amino acid in SEQ ID NO: 1 from R to P, and a nucleic acid encoding the protein mutant was produced through a substitution of CGT at positions 121 to 123 in SEQ ID NO: 2 with CCG.

H91K was produced through a mutation of a 91st amino acid in SEQ ID NO: 1 from H to K, and a nucleic acid encoding the protein mutant was produced through a substitution of CAC at positions 271 to 273 in SEQ ID NO: 2 with AAA.

K95D was produced through a mutation of a 95th amino acid in SEQ ID NO: 1 from K to D, and a nucleic acid encoding the protein mutant was produced through a substitution of AAA at positions 283 to 285 in SEQ ID NO: 2 with GAT.

E123P was produced through a mutation of a 123rd amino acid in SEQ ID NO: 1 from E to P, and a nucleic acid encoding the protein mutant was produced through a substitution of GAG at positions 367 to 369 in SEQ ID NO: 2 with CCG.

L136F was produced through a mutation of a 136th amino acid in SEQ ID NO: 1 from L to F, and a nucleic acid encoding the protein mutant was produced through a substitution of CTG at positions 406 to 408 in SEQ ID NO: 2 with TTT.

R151F was produced through a mutation of a 151st amino acid in SEQ ID NO: 1 from R to F, and a nucleic acid encoding the protein mutant was produced through a substitution of CGT at positions 451 to 453 in SEQ ID NO: 2 with TTT.

H124E was produced through a mutation of a 124th amino acid in SEQ ID NO: 1 from H to E, and a nucleic acid encoding the protein mutant was produced through a substitution of CAC at positions 370 to 372 in SEQ ID NO: 2 with GAA.

V168Y was produced through a mutation of a 168th amino acid in SEQ ID NO: 1 from V to Y, and a nucleic acid encoding the protein mutant was produced through a substitution of GTG at positions 502 to 504 in SEQ ID NO: 2 with TAT.

C198K was produced through a mutation of a 198th amino acid in SEQ ID NO: 1 from C to K, and a nucleic acid encoding the protein mutant was produced through a substitution of TGC at positions 592 to 594 in SEQ ID NO: 2 with AAA.

T202E was produced through a mutation of a 202nd amino acid in SEQ ID NO: 1 from T to E, and a nucleic acid encoding the protein mutant was produced through a substitution of ACC at positions 604 to 606 in SEQ ID NO: 2 with GAA.

W217K was produced through a mutation of a 217th amino acid in SEQ ID NO: 1 from W to K, and a nucleic acid encoding the protein mutant was produced through a substitution of TGG at positions 649 to 651 in SEQ ID NO: 2 with AAA.

P225L was produced through a mutation of a 225th amino acid in SEQ ID NO: 1 from P to L, and a nucleic acid encoding the protein mutant was produced through a substitution of CCG at positions 673 to 675 in SEQ ID NO: 2 with CTG.

F226V was produced through a mutation of a 226th amino acid in SEQ ID NO: 1 from F to V, and a nucleic acid encoding the protein mutant was produced through a substitution of TTC at positions 676 to 678 in SEQ ID NO: 2 with GTT.

A285V was produced through a mutation of a 285th amino acid in SEQ ID NO: 1 from A to V, and a nucleic acid encoding the protein mutant was produced through a substitution of GCG at positions 853 to 855 in SEQ ID NO: 2 with GTT.

I333V was produced through a mutation of a 333th amino acid in SEQ ID NO: 1 from I to V, and a nucleic acid encoding the protein mutant was produced through a substitution of ATT at positions 997 to 999 in SEQ ID NO: 2 with GTT.

N358F was produced through a mutation of a 358th amino acid in SEQ ID NO: 1 from N to F, and a nucleic acid encoding the protein mutant was produced through a substitution of AAC at positions 1,072 to 1,074 in SEQ ID NO: 2 with TTC.

T392V was produced through a mutation of a 392nd amino acid in SEQ ID NO: 1 from T to V, and a nucleic acid encoding the protein mutant was produced through a substitution of ACC at positions 1,174 to 1,176 in SEQ ID NO: 2 with GTT.

1419K was produced through a mutation of a 419th amino acid in SEQ ID NO: 1 from I to K, and a nucleic acid encoding the protein mutant was produced through a substitution of ATT at positions 1,255 to 1,257 in SEQ ID NO: 2 with AAA.

The UGTSL2_N23E/R41P/H91K/K95D/E123P/L136F/R151F/H124E/V168Y/C198K-

-/T202E/W217K/P225L/F226V/A285V/I333V/N358F/T392V/I419K protein mutant included all of the above mutations and was named UGTSL2_M. An amino acid sequence of this protein mutant was shown in SEQ ID NO: 3. A nucleotide sequence of a nucleic acid encoding this protein mutant was shown in SEQ ID NO: 4.

A specific preparation method of recombinant expression strains for glycosyltransferase UGTSL mutants included the following steps:
S1. Polymerase chain reaction (PCR) amplification of coding genes for site-directed mutants: Through PCR amplification, a rapid mutation was conducted with a plasmid of a non-mutant strain BL21-pRSFDuet-1-AtSuSy-UGTSL as template DNA (the underlined indicated mutated bases).

Primers for a site-directed mutation N23E:
forward primer: 5'-CCGTTTCTGGAAATTGCGAAACAGCTGGCGGAT-3' (SEQ ID NO: 9); and
reverse primer: 5'-CGCAATTTCCAGAAACGGGCTGATGTGACCGTA-3' (SEQ ID NO: 10).

Primers for a site-directed mutation R41P:
forward primer: 5'-AGCACCCCGATTAACCTGGAGAGCATCATTAAGAAAATC-3' (SEQ ID NO: 11); and
reverse primer: 5'-GTTAATCGGGGTGCTGCACAGGTAGATCAGGAA-3' (SEQ ID NO: 12).

Primers for a site-directed mutation H91K:
forward primer: 5'-ACCCTGAAAAAGGCGCTGAAAATGAGCAAGCCG-3' (SEQ ID NO: 13); and
reverse primer: 5'-CGCCTTTTTCAGGGTCGGGTTCAGGTGCGGCGG-3' (SEQ ID NO: 14).

Primers for a site-directed mutation K95D:
forward primer: 5'-GCGCTGGATATGAGCAAGCCGAACTTTAGCCGT-3' (SEQ ID NO: 15); and
reverse primer: 5'-GCTCATATCCAGCGCCTTGTGCAGGGTCGGGTT-3' (SEQ ID NO: 16).

Primers for a site-directed mutation E123P:
forward primer: 5'-TGGGCGCCGCACGTTGCGAACGAACAAAACATT-3' (SEQ ID NO: 17); and
reverse primer: 5'-AACGTGCGGCGCCCACGGCTGCAGCACGTCGTA-3' (SEQ ID NO: 18).

Primers for a site-directed mutation L136F:
forward primer: 5'-GCGGGTAAATTTCTGACCAGCTGCGCGGCGGTG-3' (SEQ ID NO: 19); and
reverse primer: 5'-GGTCAGAAATTTACCCGCCGGAATGTTTTGTTC-3' (SEQ ID NO: 20).

Primers for a site-directed mutation R151F:
forward primer: 5'-TTTTTTAAGAACCCGGGCGTTGAGTTCCCGTTT-3' (SEQ ID NO: 21); and
reverse primer: 5'-GCCCGGGTTCTTAAAAAAGCTAAAGAAATAGCTGAACACCGC-3' (SEQ ID NO: 22).

Primers for a site-directed mutation H124E:
forward primer: 5'-GCGGAGGAAGTTGCGAACGAACAAAACATTCCGGCG-3' (SEQ ID NO: 23); and
reverse primer: 5'-TTCGTTCGCAACTTCCTCCGCCCACGGCTGCAGCAC-3' (SEQ ID NO: 24).

Primers for a site-directed mutation V168Y:
forward primer: 5'-CCGGAATATGAAAAGGTTAAAATCCGTGAGATTCTGGCG-3' (SEQ ID NO: 25); and
reverse primer: 5'-CTTTTCATATTCCGGCAGGTGGATCGCCGGAAA-3' (SEQ ID NO: 26).

Primers for a site-directed mutation C198K:
forward primer: 5'-CTGATGAAAACCAGCCGTACCATCGAGGCGAAA-3' (SEQ ID NO: 27); and
reverse primer: 5'-GCTGGTTTTCATCAGCATCATCTGCTTGTTACC-3' (SEQ ID NO: 28).

Primers for a site-directed mutation T202E:
forward primer: 5'-AGCCGTGAAATCGAGGCGAAATACATTGACTATTGC-3' (SEQ ID NO: 29); and
reverse primer: 5'-CTCGATTTCACGGCTGGTGCACATCAGCAT-3' (SEQ ID NO: 30).

Primers for a site-directed mutation W217K:
forward primer: 5'-CTGTGCAACAAAAAGGTGGTTCCGGTGGGTCCG-3' (SEQ ID NO: 31); and
reverse primer: 5'-CACCTTTTTGTTGCACAGTTCGGTGCAATAGTC-3' (SEQ ID NO: 32).

Primers for a site-directed mutation P225L:
forward primer: 5'-GGTCCGCTGTTCCAAGATCTGATCACCAACGAT-3' (SEQ ID NO: 33); and
reverse primer: 5'-TTGGAACAGCGGACCCACCGGAACCACCTTCCA-3' (SEQ ID NO: 34).

Primers for a site-directed mutation F226V:
forward primer: 5'-CCGCCGGTTCAAGATCTGATCACCAACGATGCG-3' (SEQ ID NO: 35); and
reverse primer: 5'-ATCTTGAACCGGCGGACCCACCGGAACCAC-3' (SEQ ID NO: 36).

Primers for a site-directed mutation A285V:
forward primer: 5'-TGGGTTGTTCGTTTTCCGAAAGGCGAGGAACGT-3' (SEQ ID NO: 37); and
reverse primer: 5'-AAAACGAACAACCCAGATGAAGTTCACGTTGCT-3' (SEQ ID NO: 38).

Primers for a site-directed mutation I333V:
forward primer: 5'-GGCTTCGTTAGCCACTGCGGTTGGAACAGCGCG-3' (SEQ ID NO: 39); and
reverse primer: 5'-GTGGCTAACGAAGCCACCGGTGCTCGGGTGGTT-3' (SEQ ID NO: 40).

Primers for a site-directed mutation N358F:
forward primer: 5'-CCGATCCACTTCGACCAACCGATTAACGCGAAACTGATGGT-3' (SEQ ID NO: 41); and
reverse primer: 5'-CGGTTGGTCGAAGTGGATCGGCATCGCAATGATCGGAACGC-3' (SEQ ID NO: 42).

Primers for a site-directed mutation T392V:
forward primer: 5'-GCGGAAGTTCTGAAGAGCGTGGTTACCGGC-3' (SEQ ID NO: 43); and
reverse primer: 5'-CTTCAGAACTTCCGCAATCTCGCCACGGTG-3' (SEQ ID NO: 44).

Primers for a site-directed mutation I419K:
forward primer: 5'-AAGAGCAAACGTGATGAGGAAATGGACGCGGTT-3' (SEQ ID NO: 45); and
reverse primer: 5'-CTCATCACGTTTGCTCTTCAGGTTTTTGCTGATTTCACG-3' (SEQ ID NO: 46).

S2. A PCR amplification system for a target plasmid was as follows: 10 µM forward and reverse primers: each 2 µL; dNTPMix: 1 µL; 2×Max Buffer: 25 µL; template plasmid: 1 µL; 2 U/50 µL Super-Fidelity DNA polymerase: 1 µL; and sterilized water ddH₂O: making up to 50 µL.

PCR amplification conditions for a target plasmid were as follows: pre-denaturation at 95°C for 30 s; 30 cycles (denaturation at 95°C for 15 s; annealing at 65°C for 15 s; and extension at 72°C for 7 min); thorough extension at 72°C for 5 min; and finally, holding a temperature of 16°C. A PCR amplification product was tested by agarose gel nucleic acid electrophoresis.

S3. 1 µL of an endonuclease DpnI was added to a mutant PCR amplification product. A resulting reaction system was placed at 37°C to allow a constant-temperature reaction for 1 h to 2 h and then added to *Escherichia coli* BL21 (DE3) competent cells. A resulting mixture was placed on an ice for 30 min, then subjected to a heat shock at 42°C for 45 s to 90 s, and then placed on an ice for 2 min. 600 µL of an LB medium was added, and a resulting bacterial solution was shaken on a shaker at 37°C and 200 rpm for 45 min, then totally coated evenly on a kanamycin-resistant LB plate, and cultured overnight at 37°C. Two single colonies were picked from the plate, inoculated into an LB liquid medium, and cultured for 9 h. A resulting bacterial solution was stored in a glycerin tube and sequenced. A bacterial solution with a correct sequencing result was coated on a kanamycin-resistant LB plate, activated in a culture tube, and subjected to plasmid extraction. An extracted recombinant plasmid was transformed into *Escherichia coli* BL21 (DE3).

S4. The above method was adopted to construct recombinant strains expressing the following mutants: UGTSL2_N23E, UGTSL2_R41P, UGTSL2_H91K, UGTSL2_K95D, UGTSL2_E123P, UGTSL2_L136F, UGTSL2_R151F, UGTSL2_H124E, UGTSL2_V168Y, UGTSL2_C198K, UGTSL2_T202E, UGTSL2_W217K, UGTSL2_P225L, UGTSL2_F226V, UGTSL2_A285V, UGTSL2_I333V, UGTSL2_N358F, UGTSL2_T392V, and UGTSL2_I419K.

S5. A process for acquiring recombinant strains expressing the protein mutants UGTSL2_N23E/R41P/H91K/K95D/E123P/L136F/R151F/H124E-
-/V168Y/C198K-/T202E/W217K/P225L/F226V/A285V/I333V/N358F/T392V/I419K was as follows:
   (1) With a plasmid of the strain BL21-pRSFDuet-1-AtSuSy-UGTSL as a template plasmid for the target plasmid amplification reaction system in the S2 and the primers for the N23E site-directed mutation as primers, the S2 and S3 were repeated to produce an N23E strain.
   (2) With a mutated N23E plasmid as a template for the target plasmid amplification reaction system in the S2 and the primers for the R41P site-directed mutation as primers, the S2 and S3 were repeated to produce an N23E/R41P strain.
   (3) With a mutated N23E/R41P plasmid as a template for the target plasmid amplification reaction system in the S2 and the primers for the H91K site-directed mutation as primers, the S2 and S3 were repeated to produce an N23E/R41P/H91K strain.

Similarly, the following recombinant expression strains were produced successively: a recombinant strain expressing a protein mutant UGTSL2_N23E/R41P/H91K/K95D, a recombinant strain expressing a protein mutant N23E/R41P/H91K/K95D/E123P, a recombinant strain expressing a protein mutant UGTSL2_N23E/R41P/H91_K/K95DN23E/R41P/H91_K/K95D/E123P/L136F, a recombinant strain expressing a protein mutant UGTSL2_N23E/R41P/H91K/K95D/E123P/L136F/R151F, a recombinant strain expressing a protein mutant UGTSL2_N23E/R41P/H91K/K95D/E123P/L136F/R151F/H124E, a recombinant strain expressing a protein mutant UGTSL2_N23E/R41P/H91K/K95D/E123P/L136F/R151F/H124E/V168Y,
a recombinant strain expressing a protein mutant UGTSL2_N23E/R41P/H91K/K95D/E123P/L136F/R151F/H124E/V168Y/C198K,
a recombinant strain expressing a protein mutant UGTSL2_N23E/R41P/H91K/K95D/E123P/L136F/R151F/H124E/V168Y/C198K/T202E, a recombinant strain expressing a protein mutant UGTSL2_N23E/R41P/H91K/K95D/E123P/L136F/R151F/H124E/V168Y/C198K/T202E/W217 K, a recombinant strain expressing a protein mutant UGTSL2_N23E/R41P/H91K/K95D/E123P/L136F/R151F/H124E/V168Y/C198K/T202E/W217 K/P225L, a recombinant strain expressing a protein mutant UGTSL2_N23E/R41P/H91K/K95D/E123P/L136F/R151F/H124E/V168Y/C198K/T202E/W217 K/P225L/F226V, a recombinant strain expressing a protein mutant UGTSL2_N23E/R41P/H91K/K95D/E123P/L136F/R151F/H124E/V168Y/C198K/T202E/W217 K/P225L/F226V/A285V, a recombinant strain expressing a protein mutant UGTSL2_N23E/R41P/H91K/K95D/E123P/L136F/R151F/H124E/V168Y/C198K/T202E/W217 K/P225L/F226V/A285V/I333V, a recombinant strain expressing a protein mutant UGTSL2_N23E/R41P/H91K/K95D/E123P/L136F/R151F/H124E/V168Y/C198K/T202E/W217 K/P225L/F226V/A285V/I333V/N358F, a recombinant strain expressing a protein mutant UGTSL2_N23E/R41P/H91K/K95D/E123P/L136F/R151F/H124E/V168Y/C198K/T202E/W217 K/P225L/F226V/A285V/I333V/N358F/T392V, and a recombinant strain expressing a protein mutant UGTSL2_N23E/R41P/H91K/K95D/E123P/L136F/R151F/H124E/V168Y/C198K/T202E/W217 K/P225L/F226V/A285V/1333V/N358F/T392V/1419K.

### Example 2 Acquisition of crude enzymes of UGTSL2 and mutants thereof

The recombinant expression strains carrying UGTSL2 and mutants thereof prepared in Example 1 each were coated on an LB solid plate (NaCl: 10 g/L, yeast powder: 5 g/L, peptone: 10 g/L, and agar: 20 g/L) including 50 µg/L of kanamycin, and cultured in a 37°C incubator for 12 h. The next day, single colonies were picked from the plate, placed in a culture tube with 5 mL of an LB liquid medium (including 50 µg/L of kanamycin), and cultured in a shaker at 37°C and 200 rpm for 12 h to produce a seed culture. The seed culture was inoculated into 100 mL of a TB medium (yeast powder: 25 g/L, peptone: 15 g/L, NaCl: 10 g/L, glucose: 2 g/L, and lactose: 0.5 g/L, including 50 µg/L of kanamycin) at an inoculum size of 1% (v:v), cultured in a shaker at 37°C and 200 rpm under shaking for 2 h, and then further cultured in the shaker at 25°C for 20 h to 22 h.

A resulting fermentation broth was collected and subjected to frozen centrifugation (4°C, 7,000 rpm, and 6 min). A resulting supernatant was discarded to produce a bacterial pellet, and the bacterial pellet was rinsed twice with a potassium phosphate buffer. Then an appropriate amount of a potassium phosphate buffer was added. A resulting suspension was placed in an ice-water mixture, and subjected to ultrasonic disruption with an ultrasonic disruptor under the following parameters: Φ6, 300 W, and 30 min. Then centrifugation was conducted with a refrigerated centrifuge at 4°C and 8,000 rpm for 30 min to produce a supernatant, which was a crude enzyme and stored in a 4°C freezer for later use.

An enzyme activity of the glycosyltransferase NtUGT_M was determined as follows: 1 mM of a substrate rebaudioside D, 2 mM of UDPG, and 0.5 mg of a crude enzyme were added to 3 mL of an enzyme-catalyzed reaction system, and then a potassium phosphate buffer of 100 mM and pH 7.2 was supplemented. A reaction was conducted at 30°C and 200 rpm. Sampling was conducted at 0 min, 20 min, and 30 min. Sample treatment: 500 µL of a sample was collected and inactivated for 5 min in a water bath at 95°C for high-performance liquid chromatography (HPLC) testing. Definition of an enzyme activity (U): an enzyme amount required for converting 1 µmol of a product within 1 min was defined as one enzyme activity unit. With an enzyme activity of a wild enzyme as 100%, a relative enzyme activity was calculated for other mutant enzymes.

An enzyme activity of the glycosyltransferase UGTSL2 was determined as follows: 1 mM of a substrate rebaudioside A, 2 mM of UDPG, and 0.5 mg of a crude enzyme were added to 3 mL of an enzyme-catalyzed reaction system, and then a potassium phosphate buffer of 100 mM and pH 7.2 was supplemented. A reaction was conducted at 30°C and 200 rpm. Sampling was conducted at 0 min, 20 min, and 30 min. Sample treatment: 500 µL of a sample was collected and inactivated for 5 min in a water bath at 95°C for HPLC testing. Definition of an enzyme activity (U): an enzyme amount required for converting 1 µmol of a product within 1 min was defined as one enzyme activity unit. With an enzyme activity of a wild enzyme as 100%, a relative enzyme activity was calculated for other mutant enzymes.

A half-life period was detected as follows: A wild enzyme and mutant enzymes each were incubated for different times in a 37°C water bath, and enzyme activities were determined. A half-life period curve was plotted, and a half-life period was calculated. A process for determining the enzyme activities was the same as above. Enzyme activity test results showed that the multi-point mutant UGTSL2_M exhibited an enzyme activity 690% of an enzyme activity of the wild enzyme, a half-life period extended by 9.4 h compared with the wild enzyme, and a far stronger ability to catalyze rebaudioside D than the wild enzyme. Specific data was shown in Table 1.

**Table 1 Relative enzyme activities and half-life periods of UGTSL2 and mutants thereof**

| Enzyme | Relative enzyme activity (%) | Half-life period (h) |
|---|---|---|
| UGTSL2 | 100 | 4.3 |
| UGTSL2_R41P | 145 | 6.7 |
| UGTSL2_L136F | 152 | 5.2 |
| UGTSL2_V168Y | 184 | 14.6 |
| UGTSL2_F226V | 159 | 8.8 |
| UGTSL2_N358F | 163 | 8.3 |
| UGTSL2_I419K | 78 | 6.1 |
| UGTSL2_M | 690 | 13.7 |

| | | |
|---|---|---|
| Note: Other mutants exhibited a lower enzyme activity than the UGTSL2 enzyme and were not listed here. | | |

### Example 3 Construction of a three-enzyme coupled system

In the pRSFDuet-1 vector selected in Example 1, a nucleotide sequence (SEQ ID NO: 4) for a stevia-derived glycosyltransferase UGTSL2_M was inserted to a *Nde*I/*Xho*I site and a nucleotide sequence (SEQ ID NO: 8) for an *Arabidopsis thaliana-derived* SuSy *At*SuSy was inserted to a *Nco*I/*Eco*RI site, so as to produce a recombinant expression plasmid pRSFDuet-1-*At*SuSy-UGTSL. The recombinant expression plasmid was transformed into *Escherichia coli* BL21 (DE3), and the induced expression was conducted to produce BL21(DE3)-pRSFDuet-1-*At*SuSy-UGTSL. An amino acid sequence of the *Arabidopsis thaliana*-derived SuSy AtSuSy was shown in SEQ ID NO: 7.

A double-gene expression vector pRSFDuet-1 was selected. In the double-gene expression vector, a nucleotide sequence (SEQ ID NO: 6) of a stevia-derived glycosyltransferase *Nt*UGT_M was inserted to an *Nde*I/*Xho*I site and a nucleotide sequence (SEQ ID NO: 8) of an *Arabidopsis thaliana-derived* SuSy AtSuSy was inserted to an *Nco*I/*Eco*RI site, so as to produce a recombinant plasmid pRSFDuet-1-*Nt*UGT_M-*At*SuSy. The recombinant plasmid was transformed into *Escherichia coli* BL21 (DE3), and the induced expression was conducted to produce BL21(DE3) -pRSFDuet-1-*Nt*UGT_M-*At*SuSy. An amino acid sequence of the glycosyltransferase *Nt*UGT_M was shown in SEQ ID NO: 5.

Fermentation with three enzymes: The BL21(DE3)-pRSFDuet-1-*At*SuSy-UGTSL and BL21(DE3)-pRSFDuet-1-*Nt*UGT_M-*At*SuSy strains each were coated on an LB solid medium plate (NaCl: 10 g/L, yeast powder: 5 g/L, peptone: 10 g/L, and agar: 20 g/L) including 50 µg/L of kanamycin, and cultured in a 37°C incubator for 12 h. The next day, single colonies were picked from the plate, placed in a culture tube with 5 mL of an LB liquid medium (including 50 µg/L of kanamycin), and cultured in a shaker at 37°C and 200 rpm for 12 h to produce a seed culture. The seed culture was inoculated into 100 mL of a TB medium (including 50 µg/L of kanamycin) at an inoculum size of 1% (v:v), cultured in a shaker at 37°C and 200 rpm under shaking for 2 h, and then further cultured in the shaker at 25°C for 20 h to 22 h.

A resulting fermentation broth was collected and subjected to frozen centrifugation (4°C, 7,000 rpm, and 6 min). A resulting supernatant was discarded to produce a bacterial pellet, and the bacterial pellet was rinsed twice with a potassium phosphate buffer. Then an appropriate amount of a potassium phosphate buffer was added. A resulting suspension was placed in an ice-water mixture, and subjected to ultrasonic disruption with an ultrasonic disruptor under the following parameters: Φ6, 300 W, and 30 min. Then centrifugation was conducted with a refrigerated centrifuge at 4°C and 8,000 rpm for 30 min to produce a supernatant, which was a crude enzyme and stored in a 4°C freezer for later use. As a result, a crude enzyme including UGTSL2_M and a crude enzyme including *Nt*UGT_M were obtained, which were the crude enzymes adopted in Examples 4 to 8 below.

### Example 4 Optimization of a reaction temperature for a one-pot reaction system

For UGTSL2_M, *Nt*UGT_M, and *At*SuSy, a high reaction temperature can lead to a high enzyme activity, but the inactivation of an enzyme protein is often accompanied. Therefore, the selection of an appropriate reaction temperature to balance an enzyme activity and protein inactivation is an important link in the optimization of a catalytic reaction system. A reaction system was incubated at 30°C, 33°C, 37°C, and 40°C under the same other conditions, including: rebaudioside A: 50 g/L, sucrose: 450 g/L, pH 8.0 and 100 mM sodium phosphate buffer, additional 2 mM UDP (human UDP, Shanghai Yuanye Bio-Technology Co., Ltd.), and enzyme activity ratio of UGTSL2_M (25 mU/mL) to *Nt*UGT_M (25 mU/mL): 1:1. A reaction was conducted for 48 h, and then the reaction was terminated by heating. A resulting reaction solution was analyzed by HPLC. The UGTSL2_M and *Nt*UGT_M enzymes were the crude enzymes prepared in Example 3.

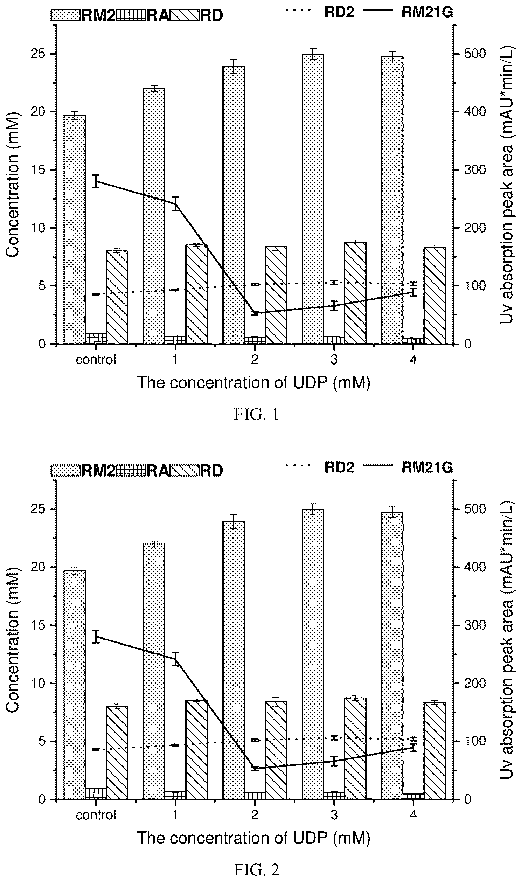

Experimental results were shown in FIG. 1. The experimental results showed that, when the reaction was conducted at 30°C and 33°C, yields of RM2 (a by-product with an unknown structure) were low and were 15.5 mM and 24.0 mM, respectively, and increased rebaudioside D and rebaudioside D2 were accumulated. Due to the reduction of catalytic efficiency at low temperatures, the two intermediates of rebaudioside D and rebaudioside D2 (RD2) could not be timely converted into the target product rebaudioside M2, and even the substrate rebaudioside A was not completely consumed. When the reaction temperature increased to 37°C and 40°C, the substrate rebaudioside A was completely converted, and yields of the synthesized rebaudioside M2 were 30.9 mM and 31.1 mM, respectively. When the reaction temperature was set to 40°C, there was the maximum yield of rebaudioside M2.

### Example 5 Optimization of a reaction pH for a one-pot reaction system

In a UGT-SuSy coupled reaction system, the catalysis was conducted at pH values of 7, 7.5, 8, 8.5, 9, and 9.5 under the same other conditions, including: rebaudioside A: 50 g/L, sucrose: 450 g/L, additional 2 mM UDP, enzyme activity ratio of UGTSL2_M (25 mU/mL) to *Nt*UGT_M (25 mU/mL): 1:1, and reaction temperature: 35°C. A catalytic reaction was conducted for 48 h, and then the catalytic reaction was terminated by heating. A resulting reaction solution was analyzed by HPLC. Experimental results were shown in FIG. 2. It could be intuitively observed that the alkaline buffer condition inhibited an efficiency of a UGT-SuSy coupled reaction. In particular, in the buffer environments of pH 9 and pH 9.5, a large amount of the substrate rebaudioside A was not converted, and yields of rebaudioside M2 were 3.36 mM and 0.58 mM, respectively. In slightly-alkaline buffer environments (pH 7.5, pH 8.0, and pH 8.5), yields of rebaudioside M2 were still insufficient and were 25.47 mM, 21.31 mM, and 9.36 mM, respectively. Considering the buffer requirements of *At*SuSy, UGTSL2_M, and *Nt*UGT_M, the buffer condition was set to a pH 7.0 100 mM sodium phosphate buffer. In this case, a yield of rebaudioside M2 was the maximum and reached 28.46 mM.

### Example 6 Optimization of an enzyme activity ratio of UGTSL2_M to NtUGT_M for a one-pot reaction system

UGTSL2_M and *Nt*UGT_M are key enzymes in the two-step glycosylation. Thus, the addition amounts and proportions of the two enzymes are closely related to the synthesis efficiencies of target and intermediate products. In the experiment of optimizing the addition amounts of UGTSL2_M and *Nt*UGT_M, the following enzyme activity ratios of UGTSL2_M to *Nt*UGT_M were set: 3:7, 4:6, 5:5, 6:4, and 7:3 (total enzyme activity: 50 mU/mL), and other conditions were the same, including: rebaudioside A: 50 g/L, sucrose: 450 g/L, additional 2 mM UDP, sodium phosphate buffer of pH 8.0 and 100 mM, and catalytic reaction temperature: 35°C. A catalytic reaction was conducted for 48 h, and then the catalytic reaction was terminated by heating. A resulting reaction solution was analyzed by HPLC.

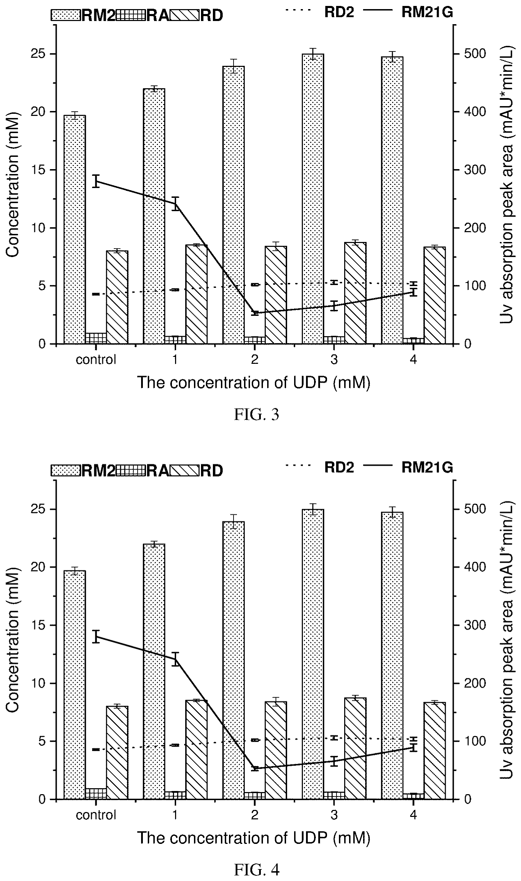

Results were shown in FIG. 3. When an enzyme activity ratio of UGTSL2_M to *Nt*UGT_M was 3:7, 4:6, 5:5, 6:4, and 7:3, yields of rebaudioside M2 were 23.72 mM, 26.1 mM, 23.08 mM, 21.96 mM, and 19.19 mM, respectively. The enzyme activity ratio of 4:6 led to the highest yield of rebaudioside M2. When the enzyme activity ratio of UGTSL2_M to *Nt*UGT_M was 4:6, the increase of either UGTSL2_M or *Nt*UGT_M could lead to the excessive accumulation of an intermediate product.

### Example 7 Optimization of a proportion of a substrate sucrose in a one-pot reaction system

In the experiment of optimizing a sucrose content, sucrose was added at amounts 6, 9, 12, 15, and 18 times an amount of an acceptor substrate in the reaction system, and other conditions were the same, including: rebaudioside A: 50 g/L, additional 2 mM UDP, enzyme activity ratio of UGTSL2_M (25 mU/mL) to *Nt*UGT_M (25 mU/mL): 1:1, sodium phosphate buffer of pH 8.0 and 100 mM, and catalytic reaction temperature: 35°C. A catalytic reaction was conducted for 48 h, and then the catalytic reaction was terminated by heating. A resulting reaction solution was analyzed by HPLC. Experimental results were shown in FIG. 4. The experimental results showed that the excessive amount of sucrose in the three-enzyme coupled catalysis system would cause the reduction of a yield of rebaudioside M2, the excessive accumulation of an intermediate product rebaudioside D, and the decrease of a conversion efficiency of rebaudioside A. It was speculated that the excessive sucrose may strengthen a pathway for UGTSL2_M to convert rebaudioside A into rebaudioside D, cause the excessive precipitation of rebaudioside D, and reduce a catalytic efficiency of *Nt*UGT_M for the conversion of rebaudioside D into rebaudioside M2. When sucrose was added at amounts 6, 9, 12, 15, and 18 times the amount of the acceptor substrate, yields were 27.90 mM, 29.69 mM, 24.02 mM, 17.51 mM, and 8.04 mM, respectively. When a ratio of the substrate to the sucrose was 1:9, there could be the maximum yield of rebaudioside M2.

### Example 8 Optimization of a proportion of a substrate UDP in a one-pot reaction system

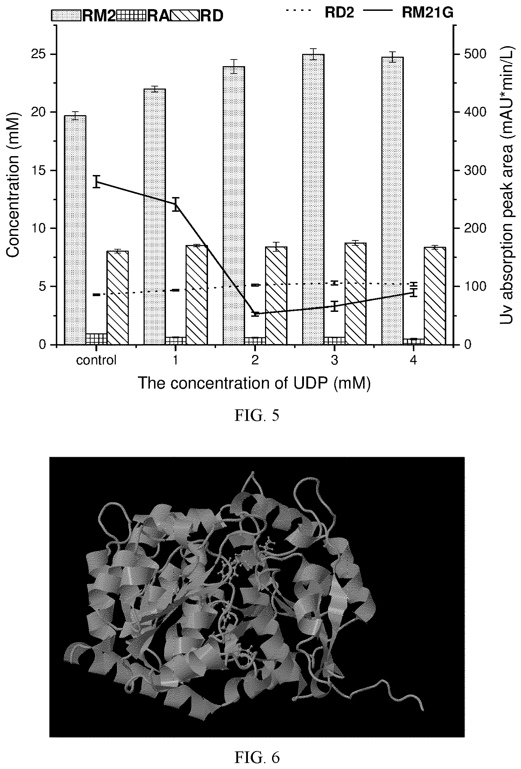

UDP included in a catalysis system comes from a disruption solution of host cells. If there is an insufficient UDP content in the disruption solution, a regeneration rate of UDPG will be limited, and the addition of a small amount of UDP can solve this problem. In the experiment of exploring an addition amount of UDP, UDP was added at 0 mM, 1 mM, 2 mM, 3 mM, and 4 mM to a three-enzyme coupled catalytic reaction system, and other reaction conditions were the same, including: rebaudioside A (RA): 50 g/L, sucrose/substrate ratio: 9:1, enzyme activity ratio of UGTSL2_M (25 mU/mL) to *Nt*UGT_M (25 mU/mL): 1:1, sodium phosphate buffer of pH 8.0 and 100 mM, and catalytic reaction temperature: 35°C. A catalytic reaction was conducted for 48 h, and then the catalytic reaction was terminated by heating. A resulting reaction solution was analyzed by HPLC. Results were shown in FIG. 5. The results showed that, compared with the blank control group in which no UDP was added, the addition of UDP at 1 mM to 4 mM significantly increased a yield of RM2 in the coupled reaction, corresponding to 19.38 mM, 21.98 mM, 23.98 mM, 24.99 mM, and 24.74 mM, respectively. The addition of UDP at 3 mM led to the maximum yield of rebaudioside M2.

### Example 9 One-pot synthesis of rebaudioside M2 from rebaudioside A under catalysis

A three-enzyme coupled reaction system including 70 g/L of rebaudioside A, 420 g/L of sucrose, 3 mM of UDP, and UGTSL2_M (20 mU/mL) and *Nt*UGT_M (30 mU/mL) in an enzyme activity ratio of 4:6 was adopted. With a sodium phosphate buffer of pH 7.0 and 100 mM and at 37°C, the substrate rebaudioside A was completely converted to produce 61.0 mM of rebaudioside M2 (78.8 g/L). A product yield was 80% or more.

### Example 10 Construction of recombinant strains expressing glycosyltransferase NtUGT mutants

A double-gene expression vector pRSFDuet-1 (this vector was published in Han Guangwei. Research on Co-expression and Immunogenicity of α, β_1, β_2, and ε toxin proteins of *Clostridium perfringens*[D]. Chinese Academy of Agricultural Sciences, 2014.) was selected. A nucleotide sequence (SEQ ID NO: 65) for a stevia-derived glycosyltransferase *NtUGT* was inserted to a *Nde*I/*Xho*I site of the above vector, and a nucleotide sequence (SEQ ID NO: 68) for an *Arabidopsis thaliana-derived* SuSy AtSUS was inserted to a *Nco*I/*Eco*RI site of the above vector, so as to produce a recombinant plasmid pRSFDuet-1-*NtUGT*-AtSUS. The recombinant plasmid was transformed into *Escherichia coli* BL21 (DE3) to construct a double-enzyme co-expression recombinant strain BL21-pRSFDuet-1-*NtUGT*-AtSUS (which was abbreviated as an NtUGT strain hereinafter). An amino acid sequence of the glycosyltransferase NtUGT was shown in SEQ ID NO: 66, and an amino acid sequence of the SuSy AtSUS was shown in SEQ ID NO: 67. A recombinant plasmid for a protein mutant was produced by substituting a nucleotide sequence for the glycosyltransferase *NtUGT* with a nucleotide sequence encoding the protein mutant. The following single-point mutants were included: NtUGT_A10V, NtUGT_F72L, NtUGT_L87P, NtUGT_L123P, NtUGT_L157P, NtUGT_Q51K, NtUGT_S400R, NtUGT_N219D, and NtUGT_G380L.

An NtUGT_A10V protein mutant was produced through a mutation of a 10th amino acid in SEQ ID NO: 66 from alanine (A) to valine (V), and a nucleic acid encoding the protein mutant was produced by substituting GCT at positions 28 to 30 in SEQ ID NO: 65 with GTT.

An NtUGT_L87P protein mutant was produced through a mutation of a 87th amino acid in SEQ ID NO: 66 from leucine (L) to proline (P), and a nucleic acid encoding the protein mutant was produced by substituting CTG at positions 259 to 261 in SEQ ID NO: 65 with CCT.

An NtUGT_G380L protein mutant was produced through a mutation of a 380th amino acid in SEQ ID NO: 66 from glycine (G) to leucine (L), and a nucleic acid encoding the protein mutant was produced by substituting GGT at positions 1,138 to 1,140 in SEQ ID NO: 65 with TTA.

An NtUGT_Q51K protein mutant was produced through a mutation of a 51st amino acid in SEQ ID NO: 66 from glutamine (Q) to lysine (K), and a nucleic acid encoding the protein mutant was produced by substituting CAG at positions 150 to 152 in SEQ ID NO: 65 with AAG.

An NtUGT_F72L protein mutant was produced through a mutation of a 72nd amino acid in SEQ ID NO: 66 from phenylalanine (F) to leucine (L), and a nucleic acid encoding the protein mutant was produced through a substitution of TTC at positions 216 to 218 in SEQ ID NO: 65 with CTT.

An NtUGT_L123P protein mutant was produced through a mutation of a 123rd amino acid in SEQ ID NO: 66 from leucine (L) to proline (P), and a nucleic acid encoding the protein mutant was produced through a substitution of CTG at positions 391 to 393 in SEQ ID NO: 65 with CCT.

An NtUGT_L157P protein mutant was produced through a mutation of a 157th amino acid in SEQ ID NO: 66 from leucine (L) to proline (P), and a nucleic acid encoding the protein mutant was produced through a substitution of TTG at positions 471 to 473 in SEQ ID NO: 65 with CCT.

An NtUGT_S400R protein mutant was produced through a mutation of a 400th amino acid in SEQ ID NO: 66 from serine (S) to arginine (R), and a nucleic acid encoding the protein mutant was produced through a substitution of TCC at positions 1,200 to 1,202 in SEQ ID NO: 65 with CGT.

An NtUGT_N219D protein mutant was produced through a mutation of a 219th amino acid in SEQ ID NO: 66 from asparagine (N) to aspartic acid (D), and a nucleic acid encoding the protein mutant was produced through a substitution of AAC at positions 657 to 659 in SEQ ID NO: 65 with GAC.

An NtUGT_ A10V/Q51K/F72L/L87P/L123P/L157P/N219D/G380L protein mutant was also included. Specifically, the above mutations except for S400R were conducted simultaneously. That is, the amino acid sequence shown in SEQ ID NO: 66 was subjected to the following mutations simultaneously: a mutation of a 10th amino acid from alanine (A) to valine (V); a mutation of a 51st amino acid from glutamine (Q) to lysine (K); a mutation of a 72nd amino acid from phenylalanine (F) to leucine (L); a mutation of a 87th amino acid from leucine (L) to proline (P); a mutation of a 123rd amino acid from leucine (L) to proline (P); a mutation of a 157th amino acid from leucine (L) to proline (P); a mutation of a 219th amino acid from asparagine (N) to aspartic acid (D); and a mutation of a 380th amino acid from glycine (G) to leucine (L). A nucleotide sequence of a nucleic acid encoding the protein mutant was shown in SEQ ID NO: 69. The increase of S400R for the multi-point mutation did not increase a catalytic effect of a mutant.

A specific preparation method of recombinant expression strains for glycosyltransferase mutants included the following steps:
S1. PCR amplification of coding genes for site-directed mutants: Through PCR amplification, a rapid mutation was conducted with a plasmid of a non-mutant strain BL21-pRSFDuet-1-*NtUGT*-AtSUS as template DNA (the underlined indicated mutated bases).

Primers for a site-directed mutation A10V were as follows: forward primer: 5'-AGGTTTTCTTGTTCCCGTGGCTGGCGTATGGT-3' (SEQ ID NO: 47); and
reverse primer: 5'-CGGGAACAAGAAAACCTTTAGTTTTGTGTGTTCAGTATCCATA- -TG-3' (SEQ ID NO: 48).

Primers for a site-directed mutation Q51K:
forward primer: 5'-CAAGAAACGTATTCCGCAAAGCTACAGCAGCA-3' (SEQ ID NO: 49); and
reverse primer: 5'-GCGGAATACGTTTCTTGATGAAGCTAAGGTTGATCGGAG-3' (SEQ ID NO: 50).

Primers for a site-directed mutation F72L:
forward primer: 5'-AGCTTCCGCAACTGCCACCGCATTACCATACC-3' (SEQ ID NO: 51); and
reverse primer: 5'-TGGCAGTTGCGGAAGCTCCGGCAGGATCAGCT-3' (SEQ ID NO: 52).

Primers for a site-directed mutation L87P:
forward primer: 5'-GCCTCACCTGAACAGCACGCTGCACAAAGCGT-3' (SEQ ID NO: 53); and
reverse primer: 5'-TGCTGTTCAGGTGAGGCGGCAGACCGTTGGTG-3' (SEQ ID NO: 54).

Primers for a site-directed mutation L123P:
forward primer: 5'-TATGCAACCTTGGACCTTTGGCGTCGCTAGTT-3' (SEQ ID NO: 55); and
reverse primer: 5'-AGGTCCAAGGTTGCATAACGTCGTAGATGATCAGA-3' (SEQ ID NO: 56).

Primers for a site-directed mutation L157P:
forward primer: 5'-ACCCTGAAGTTGAGTACCCGTTTCCGGCACTT-3' (SEQ ID NO: 57); and
reverse primer: 5'-GTACTCAACTTCAGGGTTTTTGTAGAGATGAACGAAATAGCTG-3' (SEQ ID NO: 58).

Primers for a site-directed mutation N219D:
forward primer: 5'-TATGGACTATTTGGCTGAAATCATCGAAACTCGC-3' (SEQ ID NO: 59); and
reverse primer: 5'-CAGCCAAATAGTCCATATATTTGCCCTCCAACTCACG-3' (SEQ ID NO: 60).

Primers for a site-directed mutation G380L:
forward primer: 5'-TAGAGATTTTAGTGGCCCTGGAGGTCGTGCGTGAT-3' (SEQ ID NO: 61); and
reverse primer: 5'-AGGGCCACTAAAATCTCTACCAGCAACTTGGCGTT-3' (SEQ ID NO: 62).

Primers for a site-directed mutation S400R:
forward primer: GATCGCCCGTGTGATTAAGGACGTGACCTCTGG (SEQ ID NO: 63); and
reverse primer: TAATCACACGGGCGATCTCTTCACGGTGCAGG (SEQ ID NO: 64).

S2. A PCR amplification system for a target plasmid was as follows: 10 µM forward and reverse primers: each 2 µL; dNTPMix: 1 µL; 2×Max Buffer: 25 µL; template plasmid: 1 µL; 2 U/50 µL Super-Fidelity DNA polymerase: 1 µL; and sterilized water ddH₂O: making up to 50 µL.

PCR amplification conditions for a target plasmid were as follows: pre-denaturation at 95°C for 30 s; 30 cycles (denaturation at 95°C for 15 s; annealing at 65°C for 15 s; and extension at 72°C for 7 min); thorough extension at 72°C for 5 min; and finally, holding a temperature of 16°C. A PCR amplification product was tested by agarose gel nucleic acid electrophoresis.

S3. 1 µL of an endonuclease DpnI was added to a mutant PCR amplification product. A resulting reaction system was placed at 37°C to allow a constant-temperature reaction for 1 h to 2 h and then added to *Escherichia coli* BL21 (DE3) competent cells. A resulting mixture was placed on an ice for 30 min, then subjected to a heat shock at 42°C for 45 s to 90 s, and then placed on an ice for 2 min. 600 µL of an LB medium was added, and a resulting bacterial solution was shaken on a shaker at 37°C and 200 rpm for 45 min, then totally coated evenly on a kanamycin-resistant LB plate, and cultured overnight at 37°C. Two single colonies were picked from the plate, inoculated into an LB liquid medium, and cultured for 9 h. A resulting bacterial solution was stored in a glycerin tube and sequenced. A bacterial solution with a correct sequencing result was coated on a kanamycin-resistant LB plate, activated in a culture tube, and subjected to plasmid extraction. An extracted recombinant plasmid was transformed into *Escherichia coli* BL21 (DE3).

S4. The above method was used to prepare the following strains: NtUGT_F72L, NtUGT_L123P, NtUGT_L157P, NtUGT_Q51K, NtUGT_S400R, and NtUGT_N219D.

S5. A process for acquiring a NtUGT_A10V/Q51K/F72L/L87P/L123P/L157P/N219D/G380L multi-point mutant strain was as follows:
(1) With a plasmid of a mutated NtUGT_Q51K strain as a template plasmid for the target plasmid amplification reaction system in the S2 and the primers for the A10V site-directed mutation as primers, the S2 and S3 were repeated to produce an NtUGT_A10V/Q51K plasmid.
(2) With a mutated NtUGT_A10V/Q51K plasmid as a template for the target plasmid amplification reaction system in the S2 and the primers for the F72L site-directed mutation as primers, the S2 and S3 were repeated to produce an NtUGT_A10V/Q51K/F72L plasmid.
(3) With a mutated NtUGT_A10V/Q51K/F72L plasmid as a template for the target plasmid amplification reaction system in the S2 and the primers for the L87P site-directed mutation as primers, the S2 and S3 were repeated to produce an NtUGT_A1OV/Q51K/F72L/L87P plasmid.
(4) With a mutated NtUGT_A10V/Q51K/F72L/L87P plasmid as a template for the target plasmid amplification reaction system in the S2 and the primers for the L123P site-directed mutation as primers, the S2 and S3 were repeated to produce an NtUGT_A10V/Q51K/F72L/L87P/L123P plasmid.
(5) With a mutated NtUGT_A10V/Q51K/F72L/L87P/L123P plasmid as a template for the target plasmid amplification reaction system in the S2 and the primers for the L157P site-directed mutation as primers, the S2 and S3 were repeated to produce an NtUGT_A10V/Q51K/F72L/L87P/L123P/L157P plasmid.
(6) With a mutated NtUGT_A10V/Q51K/F72L/L87P/L123P/L157P plasmid as a template for the target plasmid amplification reaction system in the S2 and the primers for the N219D site-directed mutation as primers, the S2 and S3 were repeated to produce an NtUGT_A10V/Q51K/F72L/L87P/L123P/L157P/N219D plasmid.
(7) With a mutated NtUGT_A10V/Q51K/F72L/L87P/L123P/L157P/N219D plasmid as a template for the target plasmid amplification reaction system in the S2 and the primers for the G380L site-directed mutation as primers, the S2 and S3 were repeated to produce an NtUGT_A10V/Q51K/F72L/L87P/L123P/L157P/N219D/G380L plasmid.

Relevant parameters of mutation sites were predicted and analyzed by the AMBER21 software, and results were shown in Table 2. A structure of an NtUGT protein was simulated and a spatial position of a mutant amino acid was predicted with the Aphafold2 and Rosetta software.

**Table 2 Analysis of prediction parameters for mutation sites**

| Protein mutant | Free energy change (rosetta) | | Free energy change (foldX) | Mean B factor (Å²) | Thermal stability score |
|---|---|---|---|---|---|
| NtUGT | 0 | | 0 | 67.34 | 86 |
| NtUGT_F72L | -3.41 | | -2.4 | 62.99 | 73 |
| NtUGT_L123P | -3.52 | | -1.39 | 58.76 | 93 |
| NtUGT_L157P | -2.46 | | -1.07 | 53.79 | 96 |
| NtUGT_Q51K | 0 | | 0.11 | 49.41 | 79 |
| NtUGT_S400R | -2.91 | | -1.51 | 61.62 | 93 |
| NtUGT_N219D | -2.03 | | -1.06 | 63.44 | 86 |

### Example 11 Fermentation with mutant enzymes

A recombinant strain for a single-point mutant NtUGT_Q51K of the glycosyltransferase NtUGT and a recombinant strain for a multi-point mutant NtUGT_A10V/Q51K/F72L/L87P/L123P/L157P/N219D/G380L each were coated on an LB solid medium plate including 50 µg/L of kanamycin, and cultured in a 37°C incubator for 12 h. The next day, single colonies were picked from the plate, placed in a culture tube with 5 mL of an LB liquid medium (including 50 µg/L of kanamycin), and cultured in a shaker at 37°C and 200 rpm for 12 h to produce a seed culture. The seed culture was inoculated into 100 mL of a TB medium (including 50 µg/L of kanamycin) at an inoculum size of 1% (v:v), cultured in a shaker at 37°C and 200 rpm under shaking for 2 h, and then further cultured in the shaker at 25°C for 20 h to 22 h.

A resulting fermentation broth was collected and subjected to frozen centrifugation (4°C, 7,000 rpm, and 6 min). A resulting supernatant was discarded to produce a bacterial pellet, and the bacterial pellet was rinsed twice with a potassium phosphate buffer. Then an appropriate amount of a potassium phosphate buffer was added. A resulting suspension was placed in an ice-water mixture, and subjected to ultrasonic disruption with an ultrasonic disruptor under the following parameters: Φ6, 300 W, and 30 min. Then centrifugation was conducted with a refrigerated centrifuge at 4°C and 8,000 rpm for 30 min to produce a supernatant, which was a crude enzyme and stored in a 4°C freezer for later use.

### Detection of enzyme activities and half-life periods of the wild enzyme and the mutant enzymes

An enzyme activity of the glycosyltransferase was determined as follows: 1 mM of a substrate rebaudioside D, 2 mM of UDPG, and 0.5 mg of a crude enzyme were added to 3 mL of an enzyme-catalyzed reaction system, and then a potassium phosphate buffer of 100 mM and pH 7.2 was supplemented. A reaction was conducted at 30°C and 200 rpm. Sampling was conducted at 0 min, 20 min, and 30 min. Sample treatment: 500 µL of a sample was collected and inactivated for 5 min in a water bath at 95°C for HPLC testing.

Definition of an enzyme activity (U): an enzyme amount required for converting 1 µmol of a product within 1 min was defined as one enzyme activity unit. With an enzyme activity of a wild enzyme as 100%, a relative enzyme activity was calculated for other mutant enzymes.

A half-life period was detected as follows: A wild enzyme and mutant enzymes each were incubated for different times in a 37°C water bath, and enzyme activities were determined. A half-life period curve was plotted, and a half-life period was calculated. A process for determining the enzyme activities was the same as above.

Enzyme activity determination results showed that, compared with the wild enzyme, the single-point mutant NtUGT_Q51K had an enzyme activity increased by 559% and a half-life period extended merely by 0.3 h. The multi-point mutant NtUGT_A10V/Q51K/F72L/L87P/L123P/L157P/N219D/G380L exhibited an enzyme activity 570% of an enzyme activity of the wild enzyme, a half-life period extended by 8.6 h compared with the wild enzyme, and a far stronger ability to catalyze rebaudioside D than the wild enzyme. Specific data was shown in Table 3.

**Table 3 Relative enzyme activities and half-life periods of mutants**

| Enzyme | Relative enzyme activity (%) | Half-life period (h) |
|---|---|---|
| NtUGT (wild enzyme) | 100 | 4.5 |
| NtUGT_F72L | 132 | 3.4 |
| NtUGT_L123P | 105 | 4.2 |
| NtUGT_L157P | 93 | 14.6 |
| NtUGT_Q51K | 659 | 4.8 |
| NtUGT_S400R | 63 | 8.3 |
| NtUGT_N219D | 78 | 6.1 |
| NtUGT_ A10V/Q51K/F72L/L87P/L123P/L157P/N219D/G380L | 570 | 13.1 |

| | | |
|---|---|---|
| Note: Other mutants exhibited a lower enzyme activity than the wild enzyme and were not listed here. | | |

### Example 12 Comparison of the non-mutant strain and the mutant strains to synthesize rebaudioside M2

Under the same catalytic reaction conditions, the non-mutant strain and the mutant strains were used to catalyze the synthesis of rebaudioside M2 from the substrate rebaudioside D. A sample was collected at 36 h, treated, and analyzed by HPLC.

A catalytic reaction system was as follows: rebaudioside D, sucrose, and crude enzyme: each 10 mg/mL, and potassium phosphate buffer of 100 mM and pH 7.2, where a mass ratio of the rebaudioside D to the sucrose was 1:5. A catalytic reaction was conducted at 37°C and 200 rpm. When a content of rebaudioside D was 100 g/L and a content of sucrose was 500 g/L in the catalytic reaction system, the results in Table 3 were obtained.

Results were shown in Table 4. Within 36 h of the reaction, compared with the non-mutant strain, the mutant strains led to a high yield of rebaudioside M2 during the catalytic synthesis and allowed a final rebaudioside M2 concentration of 116.7 g/L, achieving the efficient catalytic synthesis of rebaudioside M2.

**Table 4 Comparison of rebaudioside RM2 concentrations in the mutant and control groups during catalytic synthesis**

| | 12h | 24h | 36h |
|---|---|---|---|
| NtUGT | 9.8 g/L | 9.8 g/L | 9.6 g/L |
| NtUGT_F72L | 14.2 g/L | 14.3 g/L | 14.2 g/L |
| NtUGT_L123P | 25.8 g/L | 28.8 g/L | 30.9 g/L |
| NtUGT_L157P | 45.4 g/L | 64.9 g/L | 72.4 g/L |
| NtUGT_Q51K | 60.3 g/L | 72.2 g/L | 74.2 g/L |
| NtUGT_S400R | 32.8 g/L | 32.5 g/L | 31.7 g/L |
| NtUGT_N219D | 29.9 g/L | 35.6 g/L | 42.7 g/L |
| NtUGT_ A10V/Q51K/F72L/L87P/L123P/L157P/N219D/G380L | 63.5 g/L | 88.9 g/L | 116.7 g/L |

## Claims

1. A glycosyltransferase UGTSL2 mutant, wherein the glycosyltransferase UGTSL2 mutant is any one selected from the group consisting of the following (A) to (C):
(A) a protein produced through any one or more selected from the group consisting of the following mutations based on an amino acid sequence shown in SEQ ID NO: 1:
a mutation of a 23rd amino acid from N to E;
a mutation of a 41st amino acid from R to P;
a mutation of a 91st amino acid from H to K;
a mutation of a 95th amino acid from K to D;
a mutation of a 123rd amino acid from E to P;
a mutation of a 136th amino acid from L to F;
a mutation of a 151st amino acid from R to F;
a mutation of a 124th amino acid from H to E;
a mutation of a 168th amino acid from V to Y;
a mutation of a 198th amino acid from C to K;
a mutation of a 202nd amino acid from T to E;
a mutation of a 217th amino acid from W to K;
a mutation of a 225th amino acid from P to L;
a mutation of a 226th amino acid from F to V;
a mutation of a 285th amino acid from A to V;
a mutation of a 333rd amino acid from I to V;
a mutation of a 358th amino acid from N to F;
a mutation of a 392nd amino acid from T to V; and
a mutation of a 419th amino acid from I to K;
(B) a protein that has an identity of 95% or more with and the same function as an amino acid sequence defined in the (A); and
(C) a fusion protein produced by linking a tag to a terminus of the protein defined in the (A) or the (B).

2. The glycosyltransferase UGTSL2 mutant according to claim 1, wherein an amino acid sequence of the glycosyltransferase UGTSL2 mutant is shown in SEQ ID NO: 3.

3. A biological material selected from the group consisting of the following:
(A) an expression gene encoding the glycosyltransferase UGTSL2 mutant according to claim 1 or 2;
(B) a recombinant plasmid carrying the expression gene in the (A); and
(C) a recombinant cell carrying the recombinant plasmid or the expression gene encoding the glycosyltransferase UGTSL2 mutant.

4. An enzyme composition, comprising:
a glycosyltransferase UGTSL2 mutant, a glycosyltransferase *Nt*UGT_M, and a sucrose synthase (SuSy) AtSuSy, wherein the glycosyltransferase UGTSL2 mutant is the glycosyltransferase UGTSL2 mutant according to claim 1 or 2;
the glycosyltransferase *Nt*UGT_M is the following (A1) or (A2) : (A1) an amino acid sequence shown in SEQ ID NO: 5; and
(A2) a protein that has an identity of 95% or more with and the same function as the amino acid sequence defined in the (Al); and
the SuSy AtSuSy is the following (B1) or (B2): (B1) an amino acid sequence shown in SEQ ID NO: 7; and
(B2) a protein that has an identity of 95% or more with and the same function as the amino acid sequence defined in the (B1).

5. The composition according to claim 4, wherein
an enzyme activity ratio of the glycosyltransferase UGTSL2 mutant to the glycosyltransferase *Nt*UGT_M is (3-7):(7-3).

6. A complete set of recombinant strains expressing the enzyme composition according to claim 4 or 5, comprising a recombinant strain A and a recombinant strain B, wherein the recombinant strain A comprises a recombinant plasmid A, and the recombinant plasmid A is produced by co-constructing a nucleic acid encoding the glycosyltransferase UGTSL2 mutant according to claim 1 or 2 and a nucleic acid encoding the SuSy *At*SuSy into an expression vector; the recombinant strain B comprises a recombinant plasmid B, and the recombinant plasmid B is produced by co-constructing a nucleic acid encoding the glycosyltransferase *Nt*UGT_M and a nucleic acid encoding the SuSy *At*SuSy into a plasmid; and an amino acid sequence of the glycosyltransferase *Nt*UGT_M is shown in SEQ ID NO: 5.

7. The complete set of recombinant strains according to claim 6, wherein host strains comprise, but are not limited to, *Escherichia coli, Saccharomyces cerevisiae, Pichia pastoris,* or *Corynebacterium glutamicum.*

8. A method for one-pot synthesis of rebaudioside M2 from rebaudioside A under biocatalysis, comprising the following steps:
adding the rebaudioside A, sucrose, and the enzyme composition according to claim 4 or an induced expressed enzyme product of the complete set of recombinant strains according to claim 5 to a catalytic reaction system, allowing a reaction, and conducting enzyme inactivation and centrifugation to produce a supernatant, which is the rebaudioside M2, wherein the rebaudioside M2 is shown in the following formula (I):

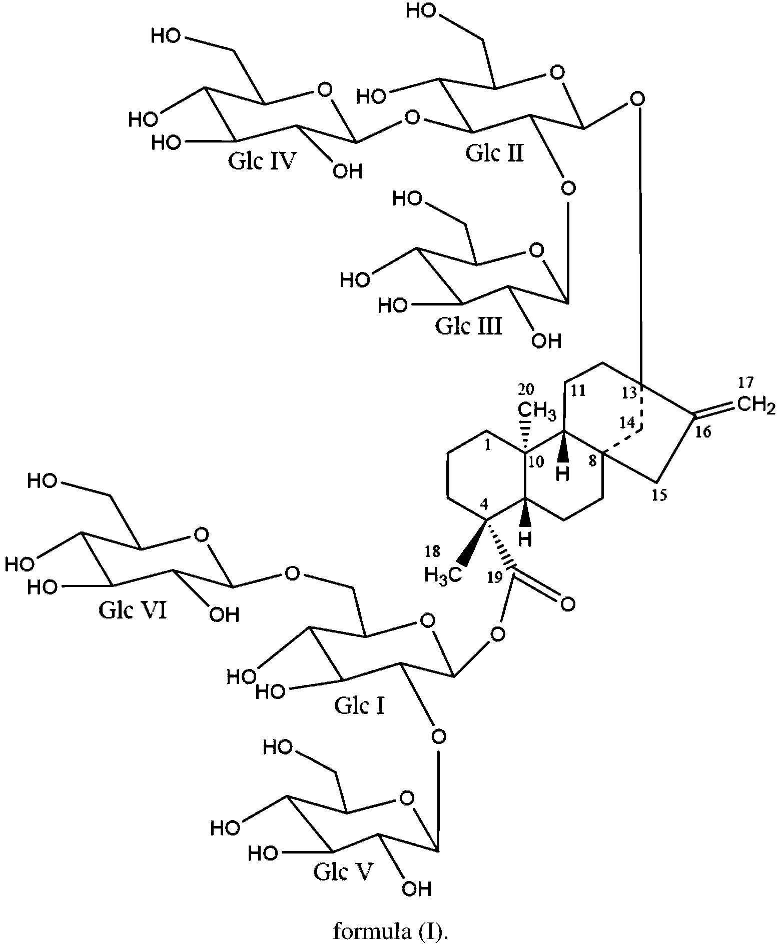

9. The method according to claim 8, wherein a process for acquiring the induced expressed enzyme product of the complete set of recombinant strains according to claim 5 or 6 comprises the following step: 1) activating the complete set of recombinant strains according to claim 5 or 6 to produce activated recombinant strains, transferring the activated recombinant strains to an induction medium, adding an inducer, and conducting an induction culture; conducting centrifugation to collect strain cells, resuspending the strain cells in an appropriate amount of a buffer, and conducting disruption; and conducting centrifugation to produce a supernatant, which is the induced expressed enzyme product; and the reaction is conducted for 5 h to 100 h.

10. The method according to claim 9, wherein a final concentration of the inducer is 0.02 g/L to 1 g/L, and the induction culture is conducted for 4 h to 50 h.

11. The method according to claim 9 or 10, wherein in the catalytic reaction system, a concentration of the rebaudioside A is 1 g/L to 1,000 g/L, a concentration of the sucrose is 1 g/L to 9,000 g/L, and an amount of the induced expressed enzyme product added is 1 MU/L to 100 MU/L; a pH of the catalytic reaction system is 7.0 to 8.5 and preferably 7.0; the reaction is conducted at 37°C to 40°C; and an enzyme activity ratio of the glycosyltransferase UGTSL2 mutant to the glycosyltransferase *Nt*UGT_M is (3-7):(7-3).

12. A glycosyltransferase mutant, wherein the glycosyltransferase mutant is the following A1) or A2):
A1) a protein produced through an amino acid residue substitution in an amino acid sequence shown in SEQ ID NO: 66, wherein the protein has an identity of 90% or more with and the same function as the amino acid sequence shown in SEQ ID NO: 66; and
A2) a fusion protein produced by linking a tag to an N-terminus and/or a C-terminus of the protein in the A1).

13. The glycosyltransferase mutant according to claim 12, wherein
the glycosyltransferase mutant is a protein produced through the following one or more amino acid residue substitutions in the amino acid sequence shown in SEQ ID NO: 66:
B1) a mutation of a 10th amino acid from alanine (A) to valine (V);
B2) a mutation of a 51st amino acid from glutamine (Q) to lysine (K);
B3) a mutation of a 72nd amino acid from phenylalanine (F) to leucine (L);
B4) a mutation of a 87th amino acid from leucine (L) to proline (P);
B5) a mutation of a 123rd amino acid from leucine (L) to proline (P);
B6) a mutation of a 157th amino acid from leucine (L) to proline (P);
B7) a mutation of a 219th amino acid from asparagine (N) to aspartic acid (D);
B8) a mutation of a 380th amino acid from glycine (G) to leucine (L); and
B9) a mutation of a 400th amino acid from serine (S) to arginine (R).

14. A biological material, wherein the biological material is any one selected from the group consisting of the following C1) to C5):
C1) a nucleic acid encoding the protein according to claim 12 or 13;
C2) an expression cassette carrying the nucleic acid in the C1);
C3) a recombinant vector carrying the nucleic acid in the C1) or a recombinant vector carrying the expression cassette in the C2);
C4) a recombinant microorganism carrying the nucleic acid in the C1), or a recombinant microorganism carrying the expression cassette in the C2), or a recombinant microorganism carrying the recombinant vector in the C3); and
C5) a transgenic plant cell line carrying the nucleic acid in the C1) or a transgenic plant cell line carrying the expression cassette in the C2).

15. A method for synthesizing rebaudioside M2 under catalysis of a glycosyltransferase mutant, comprising the following steps:
1) construction of a recombinant strain carrying a double-enzyme co-expression system: co-constructing a coding gene for the glycosyltransferase mutant according to any one of claims 12 to 13 and a SuSy gene into an expression vector to produce a recombinant plasmid, and transforming the recombinant plasmid into a host strain to produce the recombinant strain carrying the double-enzyme co-expression system;
2) inducing the recombinant strain to express the glycosyltransferase mutant and SuSy; and
3) using the two enzymes obtained in the step 2) to prepare rebaudioside M2 with rebaudioside D and sucrose as raw materials:

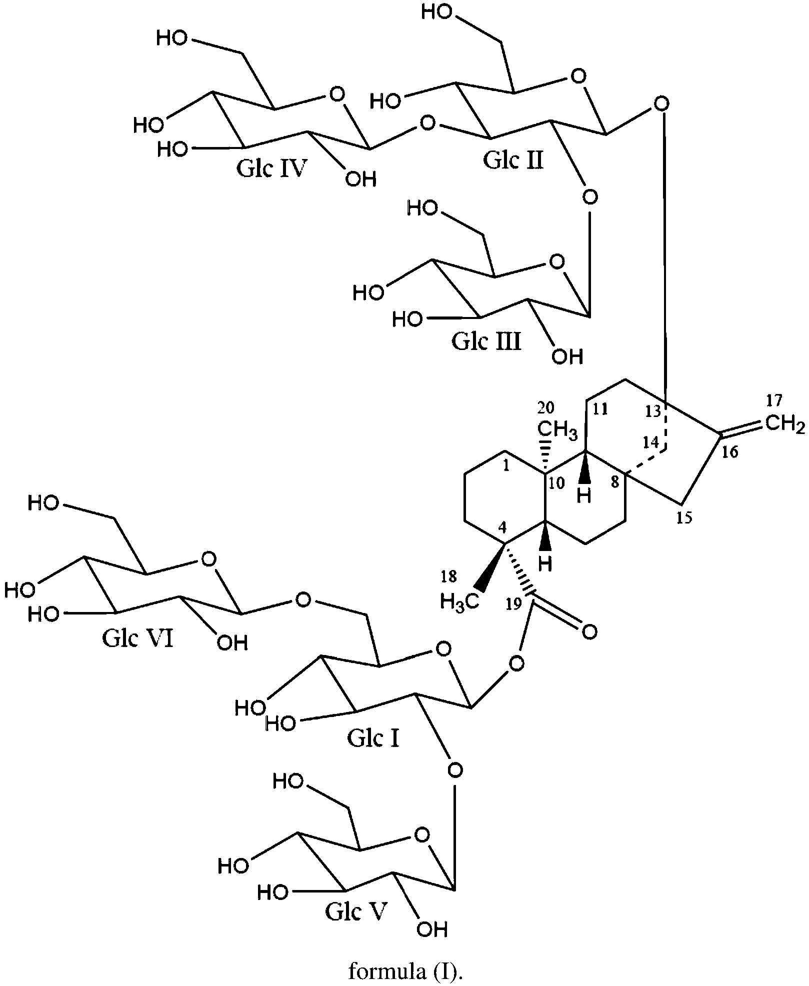

16. The method according to claim 15, wherein the expression vector is pRSFDuet-1.

17. The method according to claim 15, wherein for the induced expression in the step 2), an inducer is added at a final concentration of 0.02 g/L to 1 g/L, and induction is conducted for 4 h to 50 h.

18. The method according to claim 15, wherein the step 2) comprises: strain collection, strain disruption, and centrifugation to collect a supernatant, which is a crude enzyme.

19. The method according to claim 18, wherein
in the step 3), a concentration of the rebaudioside D is 1 g/L to 500 g/L, a concentration of the sucrose is 1 g/L to 1,500 g/L, and an amount of the crude enzyme added is 1 g/L to 100 g/L.

20. The method according to claim 15, wherein the host strain comprises, but is not limited to, *Escherichia coli, Saccharomyces cerevisiae, Pichia pastoris,* or *Corynebacterium glutamicum.*

21. The method according to any one of claims 15 to 20, wherein the rebaudioside D is synthesized with rebaudioside A as a substrate under catalysis of a glycosyltransferase.
